# EUROPEAN PATENT SPECIFICATION

(11) **EP 2 239 002 B1**
(45) Date of publication and mention of the grant of the patent: **01.05.2013**
(21) Application number: 10158226.0
(22) Date of filing: 29.03.2010
(51) Int. Cl.: A61M 15/00

(54) **Dry powder inhaler device and lock mechanism**
Trockenpulverinhalator und Sperrmechanismus
Inhalateur de poudre sèche et mécanisme de verrouillage

(30) Priority: 30.03.2009 TR 200902446; 05.05.2009 TR 200903493; 07.01.2010 TR 201000073
(43) Date of publication of application: 13.10.2010
(73) Proprietor: Sanovel Ilaç Sanayi Ve Ticaret Anonim Sirketi, 34460 Sariyer/Istanbul (TR)
(72) Inventor: Toksöz, Ahmet, 34398, Istanbul (TR); Toksöz, Zafer, 34398, Istanbul (TR); Cifter, Ümit, 34398, Istanbul (TR)
(74) Representative: Sevinç, Erkan

(56) References cited:
- EP-A1- 2 033 678
- EP-A2- 1 568 390
- WO-A1-2004/062717
- WO-A2-2005/016424

## Description

### Field of Invention

The present invention relates to a device for administering dry powder inhalation medicaments. The present invention more particularly relates to improvements conducted at the lock and blister advancement parts of dry powder inhaler devices.

### Prior Art

Diseases such as asthma, bronchitis, and COLD (Chronic Obstructive Lung Disease) substantially decrease the quality of human life, despite the developments which have been carried out in the diagnosis and therapy thereof in the recent years. It has been proposed to administer the medicaments via inhalers for optimizing the treatment of such diseases. The inhaler route of treatment is the most preferred one and it is expected to remain so, as the first option, in the future. The most important advantage of using medicaments via inhalation route is based on providing a more efficient therapy by making use of lesser medicaments, delivering higher concentration of medicaments to the airways, and particularly decreasing the systemic side effects of medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

There have been developed various inhalation devices for administering inhalation medicaments nowadays. These devices are basically classified in two groups, i.e. metered dose inhalers and dry powder inhalers. Such type of devices are structurally provided with such basic components as an actuator, counter, housing, lid, lock, etc. Additionally, powder inhalation medicaments are kept in reservoirs or containers such as blisters, capsules, etc. Blisters are structured from two basic parts, a main layer provided with cavities holding the medicament, and a strippable protective layer. Some of the currently-available dry powder inhaler devices are as follows.

One type of inhaler devices comprises an arm advancing the blister mechanism. In order to drive said mechanism, the operation is terminated with at least two forward and backward movements along the disk-shaped body. The use safety of device is ensured with an additional outer body. The use of extra devices, the necessity towards multistep operations, and the difficulties arising therefrom in such devices may cause unwanted outcomes, such as wasting the drugs due to improper use.

A pre-metered dose magazine for a dry powder inhaler is disclosed in WO 2005016424A2 wherein a dry powder inhaler including a housing having a mouthpiece and a delivery passageway connected to the mouthpiece is described. A magazine is positioned within the housing and including a plurality of reservoirs for holding doses of dry powder. The magazine is movable within the housing so that the reservoirs are sequentially positioned within the delivery passageway of the housing upon movement of the magazine.

Lock embodiments of various types have been developed so far to ensure operation safety in currently-used inhaler devices. These embodiments have aimed at preventing the unnecessary use of drugs. The applications EP1774984, US2002170560 disclose lock embodiments used in inhaler devices.

The extent of the initial operational force to be applied to a button, arm, etc. to advance the contained blister and the extent of the advancement of such elements in current blister inhaler devices are a serious problem with respect to the user. Particularly the case in which the user applies an inadequate force to this arm or button gives rise to a half opening of the blister, leading to an inadequate administration of the aimed dosage. On the contrary, an extreme force applied causes an excessive dosage to be released. In result, the need towards an embodiment which would ensure an accurate administration of the required amount of drug is obviously of vital importance.

The use of the current inhaler devices necessitates certain training and practice. The development of inhaler devices always occurs in the form of practical systems, providing the patients with improved convenience of use. Selecting the proper device for a respective patient is also an important issue. Many criteria, such as a patient's cognitive and physical efficiency, ease of use, safety and price, etc. are considered while a device is selected.

In result, there is a novelty required in the field of inhaler devices, providing high-accuracy operation, practicality, and advantages in terms of usage.

### Objects and Brief Description of Invention

The present invention relates to an improved inhaler device and lock for use with dry powder inhaling purposes, eliminating all aforesaid problems and bringing additional advantages to the relevant prior art.

Under the light of the prior art, the main object of the present invention is to provide a dry powder inhaler device, which can be operated at a desired accuracy, can peel off the blister for use, and can perform this process faultlessly as compared to precedent devices.

Another object of the present invention is to realize an inhaler device embodiment, which ensures a complete peeling-off of each medicament-carrying blister cavity whose turn comes for use, thanks to a lock mechanism having an actuating element (i.e. push element), displaceable along a determined displacement distance.

A further object of the present invention is to realize a powder inhaler device provided with a mechanism, which ensures the necessary setup for the second and subsequent uses automatically, without requiring user intervention.

Still a further object of the present invention is to avoid any unintentional use of the inhaler device, thanks to a clip, enabling the device to become locked when it is ready for use (i.e. inhalation) and the lid is in the closed position.

Yet a further object of the present invention is to provide use safety, thanks to a device which comprises a lock mechanism operating synchronously and compatibly with the advancement mechanism.

Accordingly, a novelty has been developed in the lock part of inhaler devices to achieve all objects referred to above and to emerge from the following detailed description.

In a preferred embodiment of the present invention, said novelty is realized by means of at least one lock mechanism, enabling the device to remain locked in both positions in which the device is ready for inhalation and the lid is in the closed position, and further enabling the device to setup again automatically, when the lid is closed.

In another preferred embodiment of the present invention, said lock mechanism comprises a single lock clip, which is disposed on the outer body to perform rotational motion around a pivot contact pin.

In a further preferred embodiment of the present invention, the lock clip in said lock mechanism comprises one locking side on each of the front and rear ends thereof.

Still in a further preferred embodiment of the present invention, said lock mechanism comprises a support element disposed within the interior of the lock clip.

Yet in a further preferred embodiment of the present invention, said lock mechanism is re-setup automatically, when the lid of device in the ready-for-inhalation position is closed entirely.

Yet in another preferred embodiment of the present invention, said lock mechanism comprises a push element (i.e. actuating element) that is disposed to displace axially for operating the device's blister advancement mechanism.

Still in another preferred embodiment of the present invention, said lock mechanism comprises at least one slot, which is disposed on the push element, facing (i.e. or correspond to) the locking sides of the lock clip, in order to lock the push element when it is pushed to full extent (i.e. length) at that position (i.e. pushed position), as well as to lock the same when it is released at the open position (i.e. released position).

In a further preferred embodiment of the present invention, said lock mechanism comprises at least one spring, which enables the push element to come out when it is released.

In a another preferred embodiment of the present invention, said lock/setup mechanism comprises at least one lid push surface, which separates the front locking side of the clip from the push element slot by exerting force onto the rear end of said clip, in order to enable the push element to switch from the fully-pushed locked position to the open position when the lid is closed completely.

Still in another preferred embodiment of the present invention, said lock mechanism comprises at least one guide means disposed on the inner surface of the outer body and at least one guide element guided by said guide means on the outer surface of the push element in order to control the distance said push element while it travels within the slot.

Yet in another preferred embodiment of the present invention, said lock mechanism comprises at least one circular displacement surface with a curved form, embodied on the lower surface of said lock clip, in order to enable said push element to conduct axial displacement on the lower surface of the lock clip.

Yet in a further preferred embodiment of the present invention, the spring which enables the push element in the lock mechanism to come out when it is released is a helical spring.

Still in a further preferred embodiment of the present invention, said lock mechanism comprises a spring slot on the front edge of said push element to fasten said spring.

In another preferred embodiment of the present invention, there are two guide means and two corresponding guide elements provided in said lock mechanism.

In a further preferred embodiment of the present invention, the support element in said lock mechanism is a helical spring.

Structural and characteristic features and all advantages of the present invention shall be made clear by means of annexed figures described here below and a detailed description written with references to said figures; therefore the present invention must be evaluated by taking into consideration these figures and the detailed description as well.

### Brief Description of Figures

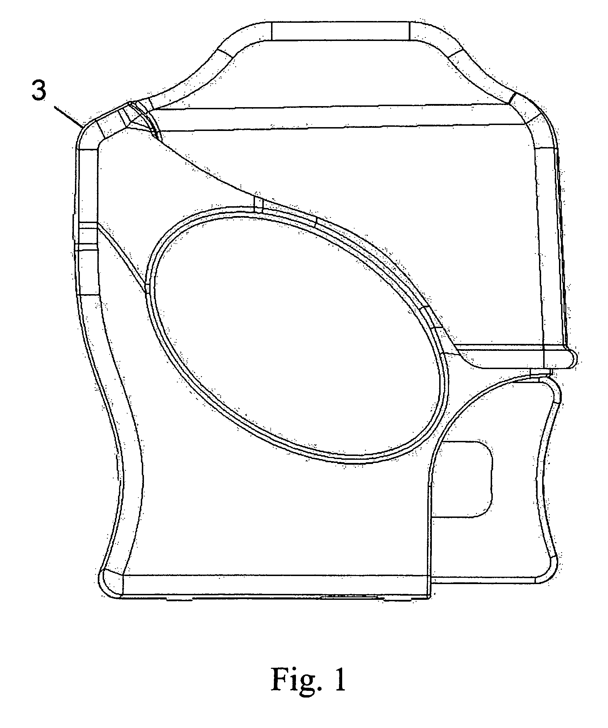

Figure 1 is an illustration of the lid of the device according to the present invention when it is in closed position (the push arm being ready to use).

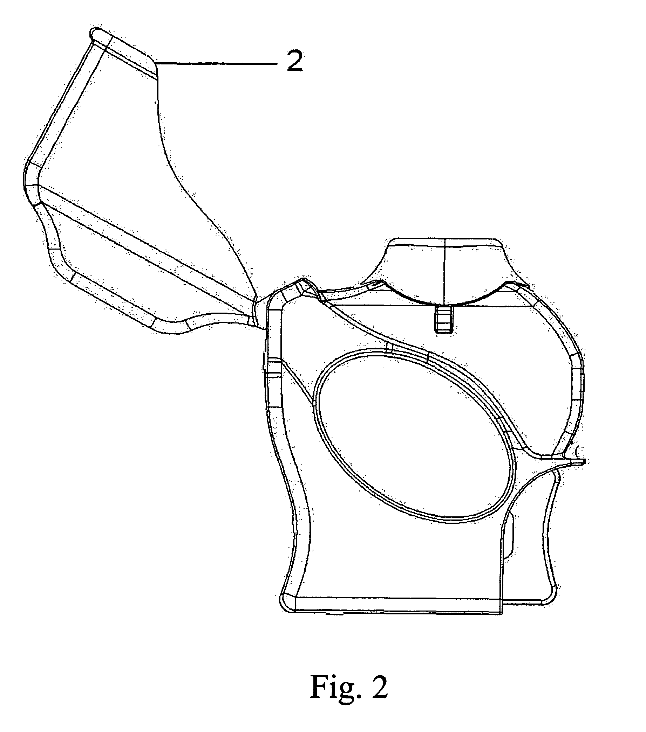

Figure 2 is an illustration of the device according to the present invention when it is ready for inhalation (the push arm being pushed completely).

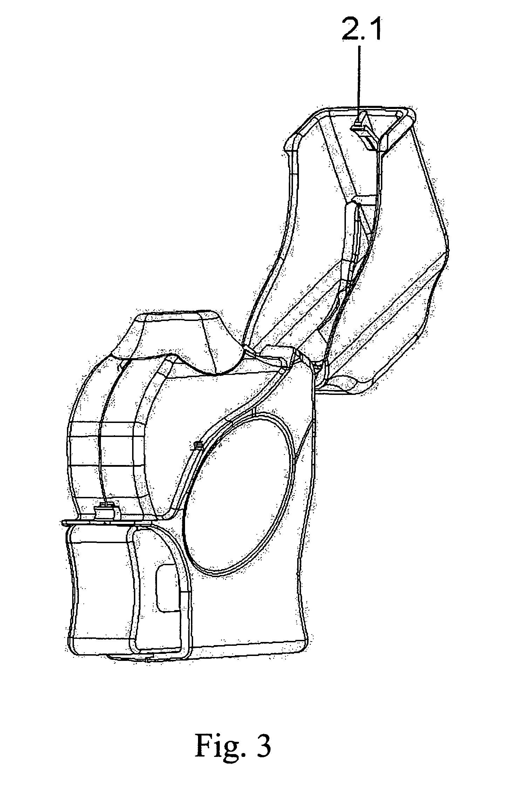

Figure 3 is an illustration of an exemplary embodiment according to the present invention.
Figure 4 is an illustration of an exemplary embodiment according to the present invention.
Figure 5 is an illustration of an exemplary embodiment according to the present invention.

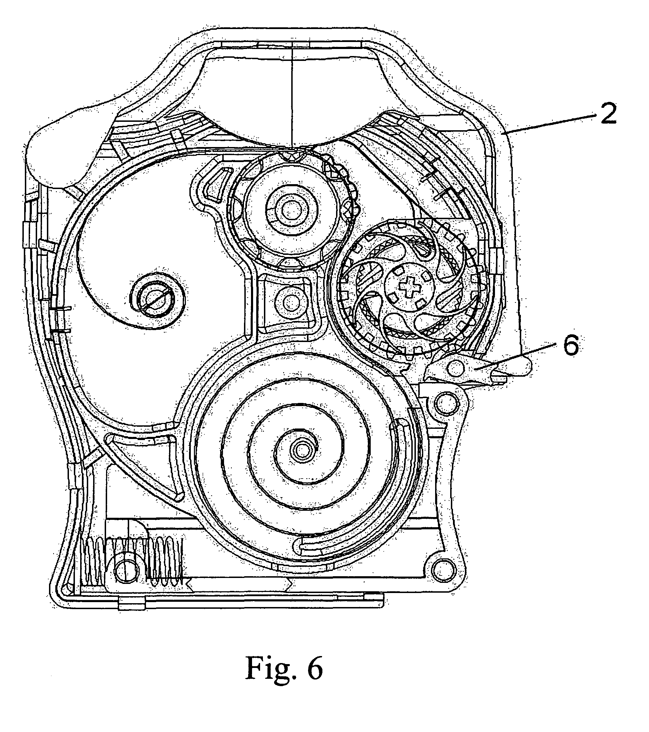

Figure 6 is an illustration of an exemplary embodiment, showing the diverging, i.e. separating clip and push element when the lid is closed.
Figure 7 is an illustration of an exemplary embodiment according to the present invention.

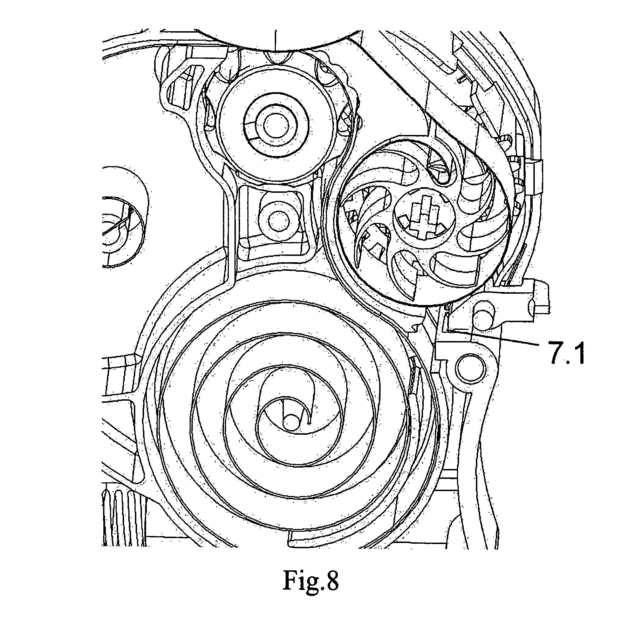

Figure 8 is an illustration of the device according to the present invention when the push arm is in the completely-pushed position.
Figure 9 is an illustration showing the disassembled state of some components of the device according to the present invention.
Figure 10 is an illustration showing the lid's interior of the device according to the present invention.
Figure 11 is an illustration of the clip according to the present invention.
Figure 12 is an illustration of the push element according to the present invention.

### Reference Numbers in Figures

| | |
|---|---|
| 1. | Blister |
| 2. | Lid |
| 2.1 | Lid push surface |
| 3. | Inhaler device |
| 4. | Contact pin |
| 5. | Support element |
| 6. | Lock clip |
| 6.1 | Front locking side |
| 6.2 | Rear locking side |
| 6.3 | Displacement surface |
| 7. | Push element |
| 7.1 | Slot |
| 7.2 | Guide |
| 7.3 | Spring slot |
| 8. | Spring |
| 9. | Outer body |
| 9.1 | Slot |
| 9.2 | Guide |
| 10 | Inner body |
| 11 | Mouthpiece |

### Detailed Description of Invention

In the following detailed description, the inhaler device (3) and the lock mechanism according to the present invention shall be described illustratively by making references to annexed figures, only to make it clear without imposing any restrictions thereon.

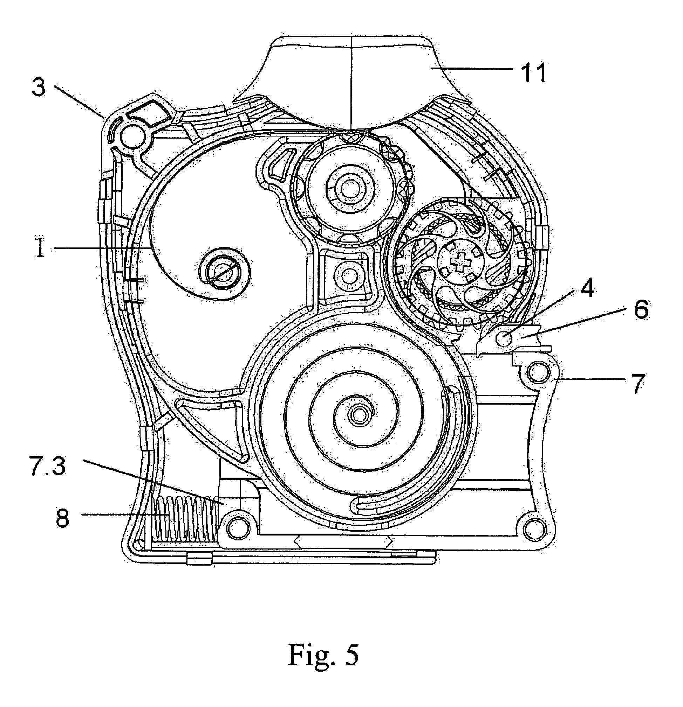

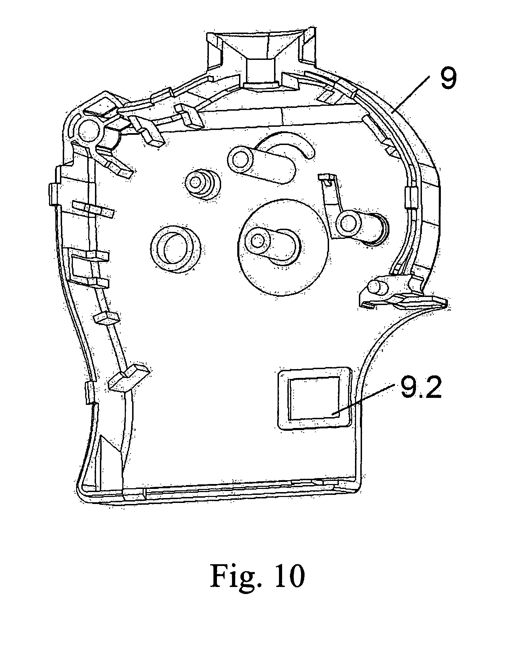

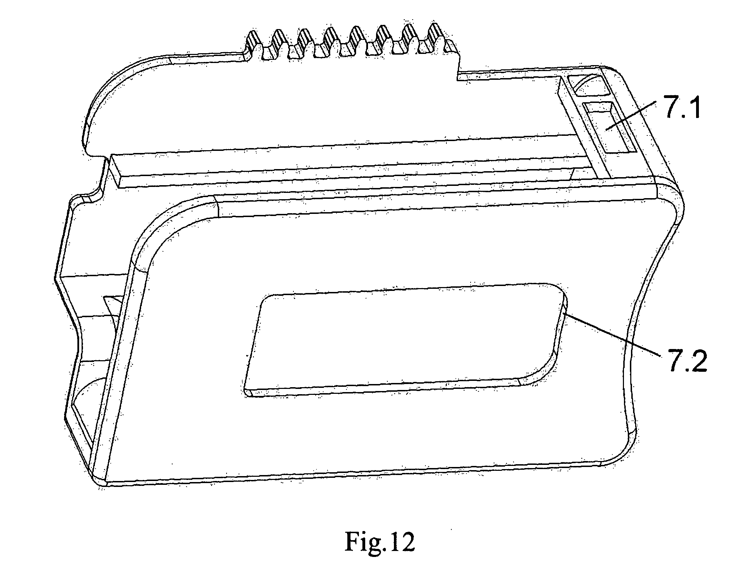

The outer body (i.e. outer housing) (9) of the inhaler device (3) according to the present invention as illustrated in figures 5, 10, and 12 is obtained by attaching two compatible parts to each other. The interior of said parts are provided with lugs (i.e. locking sides) which allow fastening the parts together by their edges. The upper part of the body is provided with a mouthpiece (11) having a medicament outlet opening. Mutually-facing guide means (9.2, 7.2) are provided in the interior of the body (9) and on the lateral surface of the push element to provide displacement orientation of said push element (7), as well as a slot (9.1) is disposed at the lower part of the body to create a displacement range to the push element.

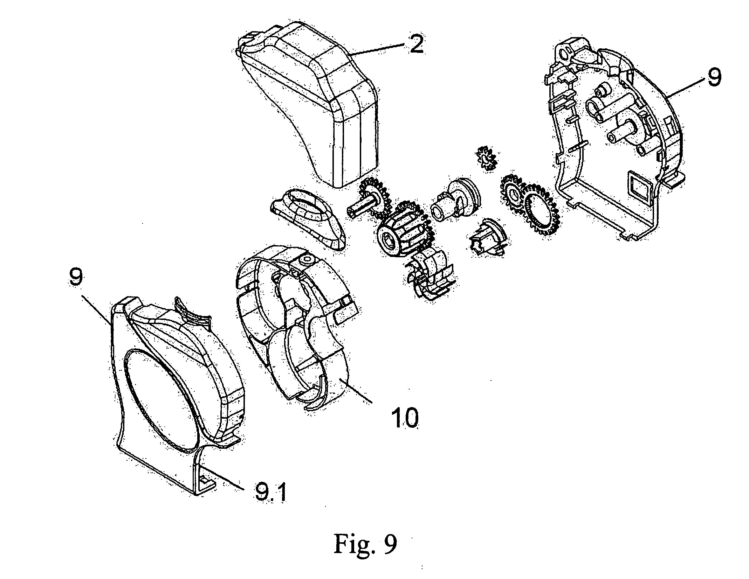

The interior of the outer body (9) is provided with an inner body (10), enabling to dispose various devices as illustrated in figures 5 and 9, and with other elements. This single-piece body comprises chambers (i.e. wells) with various volumes and recessed surfaces and locking sides. The drive mechanism and the medicament-carrying multi blister (1) are disposed within said chambers. At the beginning of the drive mechanism of said device, there is provided a push element (7), pushed or slid into the lower lateral side of said outer body (9). The upper surface of the push element (7) is provided with a side-retaining slot (7.1) and preferably a plurality of linear teeth (i.e. gear). Said push element (7) is capable to be axially displaced in the slot (9.1) at an extent, which is determined by means of a guide means (9.2) and guide element (7.2) disposed in the interior of the body (9). The grip surface provided on the exterior of the push element (7), which is the surface by which a user exerts force to push in said push element (7), is formed in an incurved manner to provide ease of use. There is provided a spring (8), disposed between said push element (7) and the body (9), this spring becoming compressed (i.e. loaded), while the push element (7) is pushed into the slot (9.1). One end of the spring is fastened in a slot (7.3), formed at the lower part of the push element.

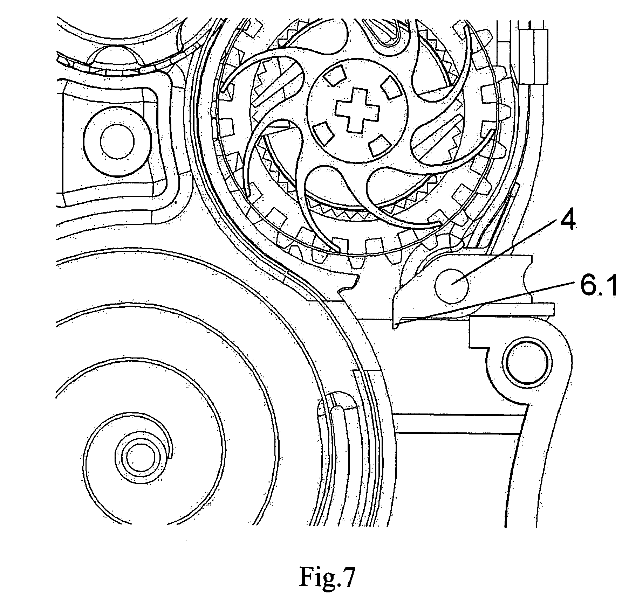

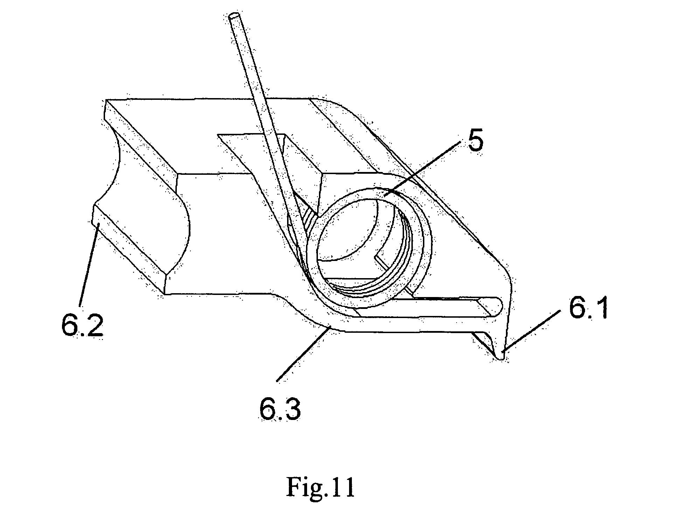

A lock clip (6) is provided on the upper part of the push element (7), as illustrated in figures 7 and 11. The clip (6) disposed here is connection with the interior of the outer body (9) by means of a pin (4), this connection being embodied to enable the pin to conduct a lever movement up- and downwards. There are provided an out-curved displacement surface (6.3), as well as locking sides (6.1, 6.2), one each at the front and rear ends, on the lower part of said clip (6). A support element (5) is disposed on a gap provided on the upper part of said clip, in order to enable the clip to maintain its current assumed position, depending on the state of the push element. This support element is a spring. This spring may either be of a helical form, or of a plate form.

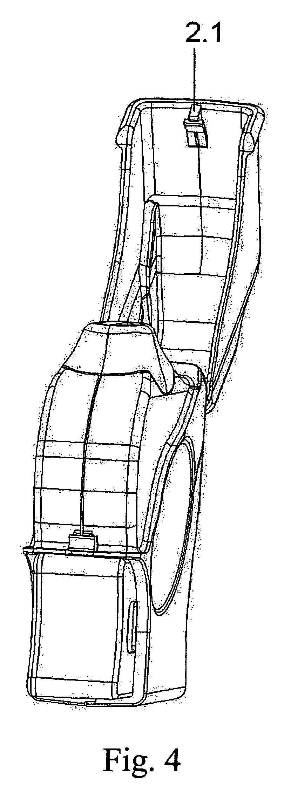

A lid push surface (2.1) is provided at the tip of the lid (2), as illustrated in figure 4.

According to the details given above, the operation of the device according to the present invention is as follows. Following the opening of lid (2), force is exerted by the user to the push element (7). Then the push element (7) is guided by means of the guides (9.2, 7.2) provided in the body (9) and on the push element itself and is slid into the slot (9.1). The linear teeth (i.e. linear gear) provided on the top of the push element (7) transmits this drive to other gears to actuate the mechanism.

As a result of sliding the push element (7) into the interior of the outer body (9), the side-retaining slot (7.1) provided on the push element (7) is coupled and fastened to the lock clip (6) front side (6.1) provided just over itself, resulting in the administration of a single dose of medicament released from the blister (1) by means of the mouthpiece. Keeping this slide-in action by the user until the locking position is achieved, guarantees the complete peeling-off of the blister (1) and ensures the accurate administration of the required dosage amount. As a result of this locking effect, the push element (7) becomes retained and it remains out-of-use for a short period of time. This slide-in action also causes the helical spring (8) to become compressed between the push element (7) and the interior of the outer body (9).

After the user inhales the powder medicament, he/she closes the lid (2), so that the push surface (2.1) provided at the tip of said lid exerts force to the rear part of the lock clip (6), thereby raising said tip above, and detaching the clip front side (6.1) and side-retaining slot (7.1) from each other. As a result of this, the spring (8) in compressed form pushes back the push element (7) and restores the device for the next use, without requiring any user intervention. When the slot (7.1) of the push element that is displaced back as far as possible is aligned with the clip rear side (6.2), they couple to each other. Thus the push element (7) becomes locked at its full-back position as well. This operation is conducted by means of a single-piece clip. It can be seen from the disclosure above, that the device according to the present invention can be operated invention can be operated by a simple single push action of the user. When the lid (2) is closed, the device (3) is set up automatically and is restored for the next use.

In consequence, an extremely-accurate and safely-operable inhaler device is obtained with the embodiment disclosed above.

The design of components used may be varied in alternative embodiments according to the type of device being produced. In result, the protection scope of the present invention is set forth in appended claims and cannot be restricted to the illustrative disclosures given above, under the detailed description. It is obvious that a person skilled in the relevant art can produce similar embodiments under the light of the foregoing disclosures, without departing from the present invention as defined by the claims.

## Claims

1. A dry powder inhaler device (3) comprising a blister (1) and a lid (2), said device (3) having at least one lock mechanism, enabling the device to remain locked in two positions, one in which the device is ready for inhalation and the second, in which the lid (2) is in the closed position, and further enabling the device (3) to setup again automatically, when the lid is closed, wherein said lock clip (6) is disposed on the outer body for performing rotational motion around a pivot contact pin (4),
**characterized in that**, said lock mechanism comprises a support element (5) disposed within the interior of a lock clip (6).

2. A dry powder inhaler device (3) according to claim 1, wherein the lock clip (6) in said lock mechanism comprises a locking side (6.1, 6.2).

3. A dry powder inhaler device (3) according to any of the preceding claims, wherein said lock mechanism is re-setup automatically, when the lid of device is closed entirely.

4. A dry powder inhaler device (3) according to any of the preceding claims, wherein said lock mechanism comprises a push element (7) that is disposed to displace axially for operating the blister advancement mechanism of the device (3).

5. A dry powder inhaler device (3) according to any of the preceding claims, wherein said lock mechanism comprises at least one slot (7.1), which is disposed on the push element, in a position corresponding to the locking sides (6.1, 6.2) of the lock clip, in order to lock the push element (7) when it is pushed to full extent, as well as to lock the same (7) when it is released to the open position.

6. A dry powder inhaler device (3) according to any of the preceding claims, wherein said lock/setup mechanism comprises at least one spring (8), which enables the push element to come out when it is released.

7. A dry powder inhaler device (3) according to any of the preceding claims, wherein said lock mechanism comprises at least one circular displacement surface (6.3) with a curved form, embodied on the lower surface of said lock clip (6), in order to enable said push element (7) to conduct axial displacement on the lower surface of the lock clip (6).

8. A dry powder inhaler device (3) according to any of the preceding claims, wherein the spring (8) in said lock mechanism is a helical spring, said spring becoming loaded with the push element (7) pushed towards the slot (9.1), and causing the push element (7) to come out when it is released.

9. A dry powder inhaler device (3) according to any of the preceding claims, wherein said lock mechanism comprises a spring slot (7.3) on the front edge of said push element (7) to fasten said spring (8).

10. A dry powder inhaler device (3) according to any of the preceding claims, **characterized in that** the support element (5) in said lock mechanism is a helical spring.

## Patentansprüche

1. Trockenpulverinhalatorvorrichtung (3) mit einem Blister (1) und einem Deckel (2), wobei die Vorrichtung (3) wenigstens einen Sperrmechanismus aufweist, der es der Vorrichtung ermöglicht, in zwei Positionen gesperrt zu verbleiben, eine, in welcher die Vorrichtung zur Inhalation bereit ist, und eine zweite, in welcher der Deckel (2) in der geschlossenen Position ist, und der es weiter der Vorrichtung (3) ermöglicht, sich wieder automatisch zu konfigurieren, wenn der Deckel geschlossen wird, wobei der Sperrclip (6) an dem äußeren Körper angeordnet ist, um eine Drehbewegung um einen Gelenkkontaktstift (4) durchzuführen,
**dadurch gekennzeichnet, dass** der Sperrmechanismus ein Trägerelement (5) umfasst, welches in dem Inneren eines Sperrclips (6) angeordnet ist.

2. Trockenpulverinhalatorvorrichtung (3) nach Anspruch 1, wobei der Sperrclip (6) in dem Sperrmechanismus eine sperrende Seite (6.1, 6.2) umfasst.

3. Trockenpulverinhalatorvorrichtung (3) nach einem der vorhergehenden Ansprüche, wobei der Sperrmechanismus automatisch neu konfiguriert wird, wenn der Deckel der Vorrichtung vollständig geschlossen wird.

4. Trockenpulverinhalatorvorrichtung (3) nach einem der vorhergehenden Ansprüche, wobei der Sperrmechanismus ein Druckelement (7) umfasst, welches für eine axiale Verlagerung angeordnet ist, um den Blistervorschubmechanismus der Vorrichtung (3) zu betätigen.

5. Trockenpulverinhalatorvorrichtung (3) nach einem der vorhergehenden Ansprüche, wobei der Sperrmechanismus wenigstens einen Schlitz (7.1) umfasst, der auf dem Druckelement in einer Position angeordnet ist, die den sperrenden Seiten (6.1, 6.2) des Sperrclips entspricht, um das Druckelement (7) zu sperren, wenn es vollständig gedrückt ist, sowie dasselbe (7) zu sperren, wenn es in die offene Position gelöst wird.

6. Trockenpulverinhalatorvorrichtung (3) nach einem der vorhergehenden Ansprüche, wobei der Sperr-/Konfigurationsmechanismus wenigstens eine Feder (8) umfasst, welche dem Druckelement ermöglicht, nach außen zu gelangen, wenn es gelöst wird.

7. Trockenpulverinhalatorvorrichtung (3) nach einem der vorhergehenden Ansprüche, wobei der Sperrmechanismus wenigstens eine runde Verlagerungsfläche (6.3) mit einer gekrümmten Form umfasst, die auf der unteren Oberfläche des Verriegelungsclips (6) ausgebildet ist, um dem Druckelement (7) zu ermöglichen, eine axiale Verlagerung an der unteren Oberfläche des Sperrclips (6) durchzuführen.

8. Trockenpulverinhalatorvorrichtung (3) nach einem der vorhergehenden Ansprüche, wobei die Feder (8) in dem Sperrmechanismus eine Spiralfeder ist, wobei die Feder gespannt wird, wenn das Druckelement (7) in Richtung des Schlitzes (9.1) gedrückt wird, und ein Herauskommen des Druckelements (7) bewirkt, wenn es gelöst wird.

9. Trockenpulverinhalatorvorrichtung (3) nach einem der vorhergehenden Ansprüche, wobei der Verriegelungsmechanismus einen Federschlitz (7.3) auf der Vorderkante des Druckelements (7) umfasst, um die Feder (8) zu befestigen.

10. Trockenpulverinhalatorvorrichtung (3) nach einem der vorhergehenden Ansprüche, **dadurch gekennzeichnet, dass** das Trägerelement (5) in dem Sperrmechanismus eine Spiralfeder ist.

## Revendications

1. Dispositif inhalateur de poudre sèche (3) comprenant un blister (1) et un couvercle (2), ledit dispositif (3) comportant au moins un mécanisme de verrouillage, permettant au dispositif de rester verrouillé dans deux positions, l'une dans laquelle le dispositif est prêt pour l'inhalation et la seconde, dans laquelle le couvercle (2) est en position fermée, et permettant en outre de régler à nouveau le dispositif (3) automatiquement, lorsque le couvercle est fermé, dans lequel ledit clip de verrouillage (6) est disposé sur le corps extérieur afin d'effectuer un mouvement de rotation autour d'un axe de pivotement de contact (4),
**caractérisé en ce que** ledit mécanisme de verrouillage comprend un élément de support (5) disposé à l'intérieur d'un clip de verrouillage (6).

2. Dispositif inhalateur de poudre sèche (3) selon la revendication 1, dans lequel le clip de verrouillage (6) dans ledit mécanisme de verrouillage comprend un côté de verrouillage (6.1, 6.2).

3. Dispositif inhalateur de poudre sèche (3) selon l'une quelconque des revendications précédentes, dans lequel ledit mécanisme de verrouillage est reconfiguré automatiquement, lorsque le couvercle du dispositif est entièrement fermé.

4. Dispositif inhalateur de poudre sèche (3) selon l'une quelconque des revendications précédentes, dans lequel ledit mécanisme de verrouillage comprend un élément de poussée (7) qui est disposé pour se déplacer axialement afin d'actionner le mécanisme d'avancement de blister du dispositif (3).

5. Dispositif inhalateur de poudre sèche (3) selon l'une quelconque des revendications précédentes, dans lequel ledit mécanisme de verrouillage comprend au moins une fente (7.1), qui est disposée sur l'élément de poussée, dans une position correspondant aux côtés de verrouillage (6.1, 6.2) du clip de verrouillage, pour verrouiller l'élément de poussée (7) lorsqu'il est poussé à pleine mesure, ainsi que pour verrouiller ce même élément (7) lorsqu'il est libéré à la position ouverte.

6. Dispositif inhalateur de poudre sèche (3) selon l'une quelconque des revendications précédentes, dans lequel ledit mécanisme de verrouillage/réglage comprend au moins un ressort (8), lequel permet à l'élément de poussée de sortir quand il est relâché.

7. Dispositif inhalateur de poudre sèche (3) selon l'une quelconque des revendications précédentes, dans lequel ledit mécanisme de verrouillage comprend au moins une surface de déplacement circulaire (6.3) ayant une forme courbe, réalisée sur la surface inférieure dudit clip de verrouillage (6), afin de permettre audit élément de poussée (7) d'effectuer un déplacement axial sur la surface inférieure du clip de verrouillage (6).

8. Dispositif inhalateur de poudre sèche (3) selon l'une quelconque des revendications précédentes, dans lequel le ressort (8) dans ledit mécanisme de verrouillage est un ressort hélicoïdal, ledit ressort étant chargé avec l'élément de poussée (7) poussé vers la fente (9.1) et amenant l'élément de poussée (7) à sortir quand il est relâché.

9. Dispositif Inhalateur de poudre sèche (3) selon l'une quelconque des revendications précédentes, dans lequel ledit mécanisme de verrouillage comprend une fente de ressort (7.3) sur le bord avant dudit élément de poussée (7) pour fixer ledit ressort (8).

10. Dispositif inhalateur de poudre sèche (3) selon l'une quelconque des revendications précédentes, **caractérisé en ce que** l'élément de support (5) dans ledit mécanisme de verrouillage est un ressort hélicoïdal.
